# EUROPEAN PATENT SPECIFICATION

(11) **EP 2 540 300 B1**
(45) Date of publication and mention of the grant of the patent: **15.07.2015**
(21) Application number: 11744815.9
(22) Date of filing: 22.02.2011
(51) Int. Cl.: A61K 31/702, A61K 33/06, A61P 37/08

(54) **EPITHELIAL CELL-CELL ADHESION ENHANCER FOR USE IN AMELIORATING, TREATMENT AND PREVENTION OF ALLERGIC DISEASES**
EPITHELZELLEN-ZELLADHÄSIONVERSTÄRKER FÜR DIE VERWENDUNG ZUR LINDERUNG, HEILUNG UND PRÄVENTION VON ALLERGISCHEN ERKRANKUNGEN
ACTIVATEUR D'ADHÉSION CELLULE-CELLULE ÉPITHÉLIALE POUR L'UTILISATION DANS LA AMÉLIORATION, TRAITMENT ET PRÉVENTION DES MALADIES ALLERGIQUE

(30) Priority: 22.02.2010 JP 2010036698
(43) Date of publication of application: 02.01.2013
(73) Proprietor: B Food Science Co., Ltd., Aichi, 478-0046 (JP)
(72) Inventor: KOGA, Yasuhiro, Isehara-shi Kanagawa 259-1143 (JP); SUZUKI, Yoshimitsu, Iga-shi Mie 518-1417 (JP); MAKISHIMA, Satoshi, Tokyo 101-0054 (JP); OGASA, Kazuo, Tokyo 101-0054 (JP); SUZUKI, Masayuki, Chita-shi Aichi 478-0046 (JP); IIZUKA, Toshiko, Chita-shi Aichi 478-0046 (JP)
(74) Representative: Prüfer & Partner GbR European Patent Attorneys
(86) International application number: PCT/JP2011/053833
(87) International publication number: WO 2011/102529

(56) References cited:
- EP-A1- 1 878 738
- WO-A1-2006/115136
- JP-A- 8 157 379
- JP-A- 2008 115 169
- JP-A- 2009 173 548
- US-A1- 2005 118 234
- GIACOMO CARLO STURNIOLO ET AL: "Effect of zinc supplementation on intestinal permeability in experimental colitis", JOURNAL OF LABORATORY AND CLINICAL MEDICINE, vol. 139, no. 5, 1 May 2002 (2002-05-01), pages 311-315, XP055069504, ISSN: 0022-2143, DOI: 10.1067/mlc.2002.123624
- MINEO, H. ET AL.: 'Indigestible disaccharides open tight junctions and enhance net calcium, magnesium, and zinc absorption in isolated rat small and large intestinal epithelium' DIG DIS SCI vol. 49, no. 1, 2004, pages 122 - 132
- AKIHIRO UCHIZONO ET AL.: 'OP-26. Allergic Rhinitis ni Taisuru Oligosaccharide (Hokuren Kestose) Toyo no Koka' MEN'EKI ALLERGY vol. 27, no. 2, 2009, pages 154 - 155

## Description

### Technical Field

The present invention relates to an epithelial cell-cell adhesion enhancer and an ameliorating, therapeutic, or prophylactic agent for allergic diseases using the same. The present invention particularly relates to an epithelial cell-cell adhesion enhancer capable of ameliorating, treating, and preventing allergic diseases by enhancing cell-cell adhesion in epithelial cells (epithelial cell-cell adhesion), which is one of the barrier functions against allergens, and an ameliorating, therapeutic, or prophylactic agent for allergic diseases using the same.

### Background Art

Allergic diseases are considered to be caused by abnormal reactions of the immune system to foreign bodies. In the human body, the organs that mainly encounter foreign substances in the external environment are the epidermis, respiratory organs, and digestive organs.

Among these organs, the digestive organs have a unique immune system, which have made them the focus of attention lately as immune organs that have a tremendous impact on the systemic immune system as well.

At present, the first-line choice for treatment of allergic diseases is synthetic oral steroid medicines, which have a mechanism of inhibiting the expression of inflammatory cytokines, which is caused by binding of allergens to the intranuclear glucocorticoid receptor. However, synthetic oral steroid medicines are reported to cause side effects such as induction of infectious diseases, atherosclerotic lesion, adrenal insufficiency, gastrointestinal disturbance, and also, menstrual abnormality in women. When serious side effects are observed, other therapeutic approaches, a concomitant treatment, or the like should also be taken into consideration.

Indeed, recently, a number of reports have been made about achievement of certain therapeutic efficacy not only by synthetic oral steroid medicines, but also by improving the balance of immunocompetent cells in the intestinal tract, and also by combining various methods for activating the immune cells.

Conventionally, as the prophylactic method for allergic diseases, based on the fact that children who have developed allergic diseases have a fewer number of enteric bifidobacteria than do healthy children, a method of attempting an improvement in the composition balance of intestinal flora has been the mainstream.

As a method based on the idea of ameliorating or preventing allergic diseases by establishing a good composition balance of intestinal flora, Japanese Patent Laid-Open No. 8-157379 (Patent Literature 1) describes a method using a prophylactic agent for allergic diseases containing a fructo-oligosaccharide. Japanese Patent Laid-Open No. 2003-201239 (Patent Literature 2) describes an immunoactivating food product containing a fructo-oligosaccharide. Also, Japanese Patent No. 4162147 (Patent Literature 3) and Japanese Patent Laid-Open No. 2008-280354 (Patent Literature 4) describe a method for inhibiting allergic diseases.

Patent Literature 1 describes a method including ingesting a composition containing a magnesium source, a fructo-oligosaccharide, and an indigestible sugar alcohol for aggravation of allergic diseases due to magnesium deficiency. This is a prophylactic method for aggravation of allergic diseases by promoting the absorption of magnesium by an indigestible sugar alcohol and an organic acid, which is produced by enteric bacteria utilizing the fructo-oligosaccharide. However, although this method can treat aggravation of allergic diseases due to magnesium deficiency, it is incapable of treating allergic diseases attributable to other causes, and thus the patients are limited. Patent Literature 2 describes a method for activating gut immunity by inducing IgA production from Peyer's patch by ingestion of a fructo-oligosaccharide. However, according to this method, the effective dose is so high that daily ingestion is very difficult.

Patent Literature 3 describes an allergic disease-inhibiting agent in which 1-kestose is the first composition accounting for the highest composition ratio in a fructo-oligosaccharide and Patent Literature 4 describes an allergic disease-inhibiting oligosaccharide in which 1-kestose is the first composition accounting for the highest composition ratio in a fructo-oligosaccharide. Compared to the effect exerted on allergic diseases described in Patent Literatures 1 and 2, the above allergic disease-inhibiting agent and allergic disease-inhibiting oligosaccharide have a higher inhibitory effect on allergic diseases. Meanwhile, Clinical effects of kestose, a prebiotic oligosaccharide, on the treatment of atopic dermatitis in infants (Non Patent Literature 1) has reported that, as a result of a study of 1-kestose ingestion in infants, no strong correlation has been observed between improvement in allergic symptoms and proliferation of bifidobacteria.

Recently, it has been shown that the cysteine/serine protease activity, which is commonly observed among potential allergenic substances, degrades the tight junction protein between epithelial cells, thereby weakening the epithelial cell-cell adhesion (Non Patent Literatures 2 and 3). In light of the above, a method based on the idea of ameliorating, treating, or preventing allergic diseases by preventing the invasion of allergens into the body by enhancing the epithelial cell-cell adhesion by repairing, or promoting the formation of, the tight junction protein between epithelial cells has been attempted.

So far, using a model employing the intestinal epithelial cells, a search has been conducted for a substance capable of enhancing the epithelial cell-cell adhesion, and examples thereof include a nucleic acid derived from the milt of fishes or yeast (Patent Literature 5), a peptide derived from a cellulase preparation (Patent Literature 6), monosialoganglioside 3 (Patent Literature 7), lipoteichoic acid derived from lactic acid bacteria (Patent Literature 8), and a whey protein (Patent Literature 9). All of the above have been proven to have an inhibitory effect on allergic diseases by promoting the formation of the tight junction protein between epithelial cells.

However, the substances described in Patent Literatures 5 to 8 involve complicated manufacturing processes, and further, industrial scale mass-production is extremely difficult. Meanwhile, the substance described in Patent Literature 9 can be produced easily; however, in order to attain the effect, it needs to be ingested in a large amount, which makes daily intake difficult. For the reasons described as above, a drug product prepared with the above substances that is capable of ameliorating, treating, or preventing allergic diseases has not yet been realized.

### Citation List

### Patent Literature

Patent Literature 1: Japanese Patent Laid-Open No. 8-157379
Patent Literature 2: Japanese Patent Laid-Open No. 2003-201239
Patent Literature 3: Japanese Patent No. 4162147
Patent Literature 4: Japanese Patent Laid-Open No. 2008-280354
Patent Literature 5: Japanese Patent No. 4050799
Patent Literature 6: Japanese Patent Laid-Open No. 2002-275196
Patent Literature 7: Japanese Patent No. 4034364
Patent Literature 8: Japanese Patent Laid-Open No. 2008-212006
Patent Literature 9: Japanese Patent No. 4330088

### Non Patent Literature

Non Patent Literature 1: Shibata R, et. al., Clin. Exp. Allergy, 2009 Sep, 39 (9), P1397 to 1403
Non Patent Literature 2: Wan H. et. al., J. Clin. Invest. 1999, 104, P123 to 133
Non Patent Literature 3: Runswick S, et. al., J. Allergy Clin. Immnol., 2003, 111, P704 to 713

### Summary of Invention

### Technical Problem

The present invention is based on the idea of ameliorating, treating, or preventing allergic diseases by inhibiting the invasion of allergens into the body by enhancing the epithelial cell-cell adhesion. The present inventors have found that a combination of a divalent metal cation and a specific oligosaccharide can further enhance the epithelial cell-cell adhesion in comparison with a divalent metal cation, thereby completing the following inventions. That is, an object of the present invention is to provide a composition containing a specific oligosaccharide and a divalent metal cation, which is effective for amelioration, treatment, or prevention of allergic diseases or for topical inflammatory reaction in an extremely safe and effective manner compared to synthetic oral steroid medicines, despite the fact that normally, an oligosaccharide is used as a food product and a divalent metal cation is present in the living body, and in this way these substances are familiar as non-toxic substances to the living body within the physiological concentration range.

### Solution to Problem

(1) Acting as a cell-cell adhesion enhancer for activation epithelial cells, for use in ameliorating, treatment or prevention of allergic diseases comprising 1-kestose and/or nystose and a divalent metal cation i.e. a calcium ion.
(2) The enhancer according to (1), wherein the enhancer comprises 1-kestose and calcium ion as active ingredients.
(3) The enhancer according to (2), wherein the 1-kestose is an oligosaccharide containing 1-kestose at a purity of 95 wt.% or more, and the divalent metal cation is present in one part by weight or more per 10 parts by weight of the oligosaccharide containing 1-kestose.
4) The enhancer according to any of (1) to (4), wherein the epithelial cell is an intestinal epithelial cell.

### Advantageous Effects of Invention

The epithelial cell-cell adhesion enhancer and the ameliorating, therapeutic, or prophylactic agent for allergic diseases using the same according to the present invention can inhibit the disruption of the tight junction protein between epithelial cells, which could cause the invasion of allergens into the body, and further, can enhance the epithelial cell-cell adhesion by repairing, or promoting the formation of, the tight junction protein between epithelial cells, thereby effectively enabling amelioration, treatment, and prevention of allergic symptoms.

### Brief Description of Drawings

[Figure 1] Figure 1 is a graph showing the results of measurement of chronological changes in TEER by the addition of 1-kestose and nystose in Example 1.
[Figure 2] Figure 2 is a graph showing the results of measurement of chronological changes in TEER by the addition of 1-kestose in Example 2.
[Figure 3] Figure 3 is a graph showing the results of measurement of chronological changes in TEER by the addition of 1-kestose in Example 3.
[Figure 4] Figure 4 is a graph showing the results of measurement of chronological changes in TEER by the addition of 1-kestose in Example 4.
[Figure 5] Figure 5 is a graph showing the results of measurement of chronological changes in TEER by the addition of 1-kestose in Example 4.
[Figure 6] Figure 6 is a graph showing the results of measurement of changes in the amount of substances permeated by the addition of 1-kestose, sucrose, and nystose in Example 5.
[Figure 7] Figure 7 is a graph showing the results of measurement of changes in TEER by the addition of 1-kestose in Example 6.

### Description

The present invention is defined by the claims. Hereinbelow, the epithelial cell-cell adhesion Hereinbelow, the epithelial cell-cell adhesion enhancer and the ameliorating, therapeutic, or prophylactic agent for allergic diseases using the same according to the present invention will be described in detail. The epithelial cell-cell adhesion enhancer according to the present invention is a cell-cell adhesion enhancer in epithelial cells, which contains 1-kestose and/or nystose and a calcium ion, or 1-kestose and a divalent metal cation as the active ingredients.

As described above, the cysteine/serine protease activity, which is commonly observed among potential allergenic substances, is known to degrade the tight junction protein between epithelial cells, thereby weakening the epithelial cell-cell adhesion (Non Patent Literatures 2 and 3). The epithelial cell-cell adhesion enhancer of the present invention repairs, or promotes the formation of, the tight junction protein between epithelial cells, thereby enhancing the epithelial cell-cell adhesion and inhibiting the invasion of allergens into the body. In light of the above, the epithelial cell-cell adhesion enhancer, and the ameliorating, therapeutic, or prophylactic agent for allergic diseases using the same according to the present invention can ameliorate, treat, or prevent allergic diseases.

In the present invention, examples of the epithelia cell include an absorptive epithelial cell, a keratinized epithelial cell, wet-stratified barrier epithelium, lining epithelium, an exocrine epithelial cell, an endocrine epithelial cell, an extracellular matrix secretory epithelial cell, and a contractile epithelial cell. Specific examples of the epithelial cell include an intestinal epithelial cell such as an epithelial cell of the small intestine, an epithelial cell of the large intestine, a duodenal epithelial cell, a gastric mucosal epithelial, an esophageal epithelial cell, a corneal epithelial cell, a conjunctival epithelial cell, an amniotic epithelial cell, a skin epithelial cell, and a palatal epithelial cell.

As the divalent metal cation, a calcium ion is used.

Conventionally, it has been shown that the cell-cell adhesion has plasticity by so-called calcium switch, which involves extracellular removal and addition of calcium ions (Sarah L. D. et. al., B. B. A., 2008, 1778, P2318 to 2324; Georgina C. et. al., J. Membrane Biol., 2010, 237, P115 to 123). Recently, it has been shown that a divalent metal cation other than a calcium ion, for example a magnesium ion and a zinc ion, also positively affects the formation of the cell-cell adhesion within the physiological concentration range (Sarah L. D. et. al., B. B. A., 2008, 1778, P2318 to 2324; Georgina C. et. al., J. Membrane Biol., 2010, 237, P115 to 123), and as a common cellular function in the above phenomena, the presence of a cellular function mediated by a divalent metal cation that has a similar effect to a calcium ion has been suggested. Accordingly, in the present invention, a phrase that 1-kestose and/or nystose and a calcium ion, or 1-kestose and a calcium ion exhibit promotion of the formation of the cell-cell adhesion is synonymous with a phrase that 1-kestose and/or nystose and a divalent metal cation, or 1-kestose and a divalent metal cation exhibit promotion of the formation of the cell-cell adhesion.

Examples of the oligosaccharide that art used in the present invention include a fructo-oligosaccharide such as 1-kestose and/or nystose. In the present Examples both 1-kestose and nystose are used.

1-Kestose is a fructotrisaccharide composed of one molecule of glucose residue and two molecules of fructose residues. Examples of a preferred form of 1-kestose that can be used in the present invention include an oligosaccharide containing 1-kestose at a purity of 95 wt.% or more. This oligosaccharide containing 1-kestose is obtained by carrying out an enzymatic reaction using sucrose as a starting material, and from the product,thus prepared, removing monosaccharides and sucrose by chromatographic separation to increase the purity of 1-kestose, and then applying a crystallization method. The oligosaccharide containing 1-kestose obtained as above has excellent solubility and is indigestible, and has low energy. Further, the above oligosaccharide containing 1-kestose has an IgA production-enhancing action and an IgE production-inhibiting action (Patent Literatures 3 and 4).

Also, as the aforementioned method of preparing a product by an enzymatic reaction using sucrose as a starting material, the method described in Japanese Patent Laid-Open No. 58-201980 can be used; as the aforementioned method for increasing the purity by removing monosaccharides and sucrose by chromatographic separation, the method described in Japanese Patent Application No. 11-34787 (Japanese Patent Laid-Open No. 2000-232878) can be used; and as the aforementioned crystallization method, the method described in Japanese Patent Application No. 2-224312 (Japanese Patent Laid-Open No. 4-235192) can be used. The contents of Japanese Patent Application No. 11-34787, Japanese Patent Application No. 2-224312, and Japanese Patent Application No. 2005-371005 (Cited Literature 3), and Japanese Patent Application No. 2008-166463 (Cited Literature 4) are encompassed by the present specification.

The daily dose of 1-kestose according to the present invention is preferably 0.015 g/kg body weight or more. In infants, the daily dose of 1-kestose is preferably approximately 2.5 g, which is obtained by conversion based on a ratio of one part by weight of divalent metal ion per 10 parts by weight of 1-kestose.

Also, the epithelial cell-cell adhesion according to the present invention is achieved by repairing, or promoting the formation of, the tight junction protein between epithelial cells.

The term "enhance" in the present invention is used interchangeably with the terms such as "activate", "promote", "strengthen", and "increase."

Examples of allergic diseases include atopy, allergic rhinitis, allergic conjunctivitis, allergic gastroenteritis, bronchial asthma, childhood asthma, urticaria, animal allergy, food allergy, metal allergy, drug allergy, and anaphylaxis. In the present invention, preferably, atopy, allergic rhinitis, allergic conjunctivitis, and food allergy are targeted.

For formulation of the epithelial cell-cell adhesion enhancer and the ameliorating, therapeutic, or prophylactic agent for allergic diseases using the same according to the present invention, a method publicly known among those skilled in the art can be used. The dosage form can also be appropriately selected by those skilled in the art. Examples of the dosage form include, when prepared as an orally administered drug, a tablet, a granule, a powder, a capsule, a coated drug, a liquid, and a suspension, and when prepared as a parenterally administered drug, an inhalant, an injection, a drip , infusion liquid, a suppository, a liniment, a spray, and a patch, and the epithelial cell-cell adhesion enhancer and the ameliorating, therapeutic, or prophylactic agent for allergic diseases using the same according to the present invention are preferably applied to the area of skin affected by pruritus or eruption, or administered to the mucus of the nose or eye. Also, the dose of the epithelial cell-cell adhesion enhancer and the ameliorating, therapeutic, or prophylactic agent for allergic diseases using the same according to the present invention can be appropriately set according to the drug formulation, administration method, and purpose of administration of the pharmaceutical composition as well as the age, body weight, and symptoms of the individual to whom the pharmaceutical composition is administered.

Hereinbelow, the epithelial cell-cell adhesion enhancer and the ameliorating, therapeutic, or prophylactic agent for allergic diseases using the same according to the present invention will be described with reference to Examples. It should be noted that the technical scope of the present invention is not limited to the characteristics shown by these Examples.

### Examples

### [Example 1: An experiment confirming the effect of 1-kestose on promoting recovery from decreased epithelial cell-cell adhesion caused by extracellular calcium deficiency]

In Example 1, the effect of 1-kestose on promoting recovery from decreased epithelial cell-cell adhesion caused by extracellular calcium deficiency was confirmed by an experiment using cultured cells.

The oligosaccharide containing 1-kestose used in the present Example 1 is composed of 98 wt.% 1-kestose, 1 wt.% nystose, and 1 wt.% sucrose. Namely, it is an oligosaccharide containing 1-kestose at a purity of 98 wt.%. Also, as the index for the cell-cell adhesion strength in cultured cells, transepithelial electrical resistance (TEER) was used. This measurement method utilizes a property that the stronger the epithelial cell-cell adhesion, the bigger the electrical resistance value between the upper layer and the lower layer of the cell culture medium, and this technique is also employed for a model experiment using epithelial cells and the like.

Caco-2 cells, which are the epithelial colorectal carcinoma cells, were subcultured in a 10% FBS-containing DMEM medium (GIBCO), and the 48th generation of cultured cells were cryopreserved and used for the following experiments. The Caco-2 cells were seeded in the upper layer of a Transwell (Corning Life Sciences) and cultured in an incubator in 5o CO₂ at 37°C in the presence of a 10% FBS-containing DMEM medium. Cell culture was performed until the electrical resistance between the upper layer and the lower layer of the Transwell (TEER) reached 400 to 500 Ω·cm².

The Caco-2 cells that were cultured until TEER of the Transwell reached 400 to 500 Ω·cm² were cultured for two hours after exchanging the 10% FBS-containing DMEM medium for a calcium-free DMEM medium. Subsequently, a calcium-containing DMEM medium to which an oligosaccharide containing 1-kestose, or nystose, was added at a concentration of 1 wt.% was added to the upper layer. Then, TEER was chronologically measured every half hour, and the cells were cultured up to 22 hours and then TEER was measured (1% kestose group, 1% nystose group, n = 3). As the control group, a group of cells that were cultured only in a calcium-free DMEM medium (negative control group, n = 3) and a group in which culture was performed for two hours in a calcium-free DMEM medium, and then the medium was only exchanged for a calcium-containing DMEM medium (positive control group, n = 3) were prepared, and chronological changes in TEER were compared.

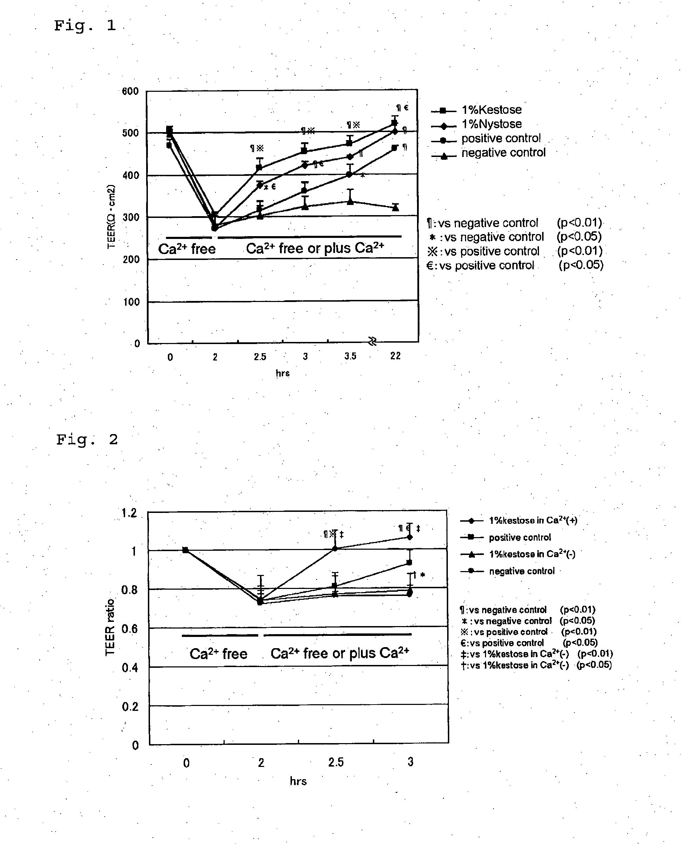

Hereinbelow, the results of measurement of TEER ratio in Example 1 are shown in Figure 1. Although recovery of TEER was hardly observed in the negative control group, significant recovery of TEER was observed in the 1% kestose group, 1% nystose group, and positive control group in comparison with the negative control group. Further, a significant recovery-promoting effect was observed in the 1% kestose group and 1% nystose group in comparison with the positive control group. Specifically, the 1% kestose group 30 minutes after exchanging a calcium-free DMEM medium for a calcium-containing DMEM medium exhibited a recovery-promoting effect 2.4 times as much as that exhibited by the positive control group on average. Also, compared to the 1% nystose group, the 1% kestose group showed a tendency of stronger promotion of recovery. These observations confirmed that 1-kestose had a strong recovery-promoting action on decreased epithelial cell-cell adhesion.

### [Example 2: An experiment confirming that the effect of 1-kestose on promoting recovery from decreased epithelial cell-cell adhesion caused by extracellular calcium deficiency is calcium dependent]

In Example 2, the effect of 1-kestose on promoting recovery from decreased epithelial cell-cell adhesion caused by extracellular calcium deficiency was confirmed to be calcium dependent by an experiment using cultured cells.

An oligosaccharide containing 1-kestose at the same purity as the one used in Example 1 was used, and the cell culture was performed under the same conditions as Example 1.

The Caco-2 cells that were cultured until TEER of the Transwell reached 400 to 500 Ω·cm² were cultured for two hours after exchanging the 10% FBS-containing DMEM medium for a calcium-free DMEM medium. Subsequently, a calcium-containing DMEM medium to which an oligosaccharide containing 1-kestose was added at a concentration of 1 wt.% was added to the upper layer. Then, TEER was chronologically measured every half hour, and the cells were cultured up to three hours and then TEER was measured (calcium (+) 1% kestose group, n = 3). Also, a group in which an oligosaccharide containing 1-kestose was added at a concentration of 1 wt.% to the calcium-free DMEM medium, while the medium was not exchanged for a calcium-containing DMEM medium, and cell culture was performed up to three hours and then TEER was measured was prepared (calcium (-) 1% kestose group, n = 3). As the control group, a group of cells that were cultured only in a calcium-free DMEM medium (negative control group, n = 3) and a group in which culture was performed for two hours in a calcium-free DMEM medium, and then the medium was only exchanged for a calcium-containing DMEM medium (positive control group, n = 3) were prepared, and chronological changes in TEER were compared.

Hereinbelow, the results of measurement of TEER ratio in Example 2 are shown in Figure 2. Recovery of TEER was hardly observed in the negative control group and the calcium (-) 1% kestose group. However, similarly to Example 1, significant recovery of TEER was observed in the calcium (+) 1% kestose group and the positive control group, and similarly to Example 1, a significant effect of promoting recovery of TEER was observed in the calcium (+) 1% kestose group also in comparison with the positive control group. These observations confirmed that 1-kestose did not exhibit an effect of recovering TEER in the absence of extracellular calcium, and a recovery-promoting effect of 1-kestose on decreased epithelial cell-cell adhesion occurred in an extracellular calcium-dependent manner.

### [Example 3: An experiment confirming that the effect of 1-kestose on promoting recovery from decreased epithelial cell-cell adhesion caused by extracellular calcium deficiency is concentration dependent]

In Example 3, the effect of 1-kestose on promoting recovery from decreased epithelial cell-cell adhesion caused by extracellular calcium deficiency was confirmed to be concentration dependent by an experiment using cultured cells.

An oligosaccharide containing 1-kestose at the same purity as the one used in Example 1 was used, and the cell culture was performed under the same conditions as Example 1.

The Caco-2 cells that were cultured until TEER of the Transwell reached 400 to 500 Ω·cm² were cultured for two hours after exchanging the 10% FBS-containing DMEM medium for a calcium-free DMEM medium. Subsequently, a calcium-containing DMEM medium to which an oligosaccharide containing 1-kestose was added at a concentration of 1 wt.% or 0.1 wt.% was added to the upper layer. Then, TEER was chronologically measured every half hour, and the cells were cultured up to 3.5 hours and then TEER was measured (1% kestose group, 0.1% kestose group, n = 3). As the control group, a group of cells cultured only in a calcium-free DMEM medium (negative control group, n = 3) and a group in which culture was performed for two hours in a calcium-free DMEM medium, and then the medium was only exchanged for a calcium-containing DMEM medium (positive control group, n = 3) were prepared, and chronological changes in TEER were compared.

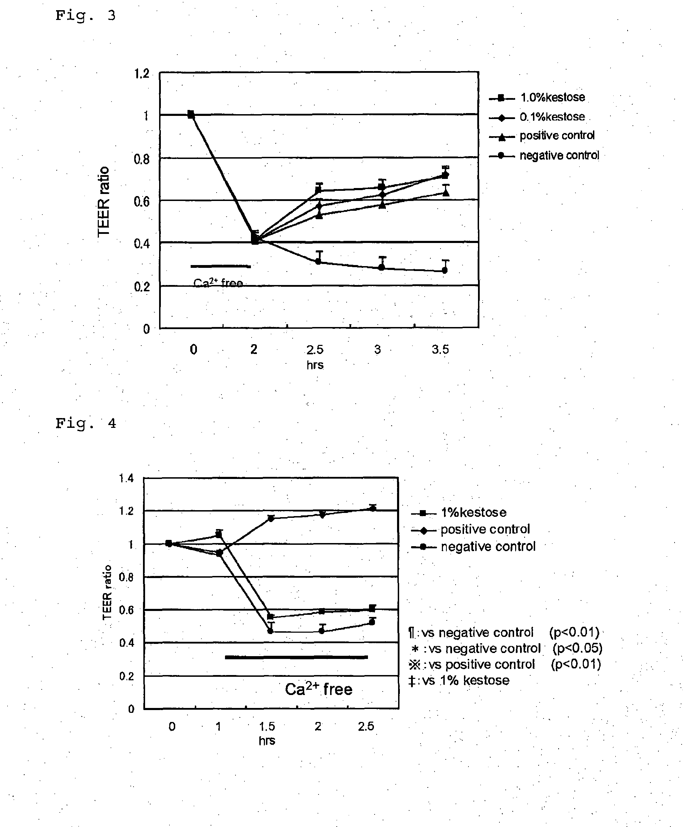

Hereinbelow, the results of measurement of TEER ratio in Example 3 are shown in Figure 3. Similarly to Example 1, recovery of TEER was hardly observed in the negative control group. However, significant recovery of TEER was observed in the 1% kestose group, 0.1% kestose group, and positive control group. Also, the 1% kestose group exhibited a tendency of stronger promotion of recovery of TEER than did the 0.1% kestose group. These observations confirmed that a recovery-promoting effect of 1-kestose on decreased epithelial cell-cell adhesion occurred in a concentration-dependent manner.

### [Example 4: An experiment examining the effect of prior administration of 1-kestose on inhibition of a decrease in the epithelial cell-cell adhesion caused by extracellular calcium deficiency]

In Example 4, whether or not a decrease in the epithelial cell-cell adhesion caused by extracellular calcium deficiency could be inhibited by prior administration of 1-kestose was examined by an experiment using cultured cells.

An oligosaccharide containing 1-kestose at the same purity as the one used in Example 1 was used, and the cell culture was performed under the same conditions as Example 1.

The Caco-2 cells were cultured until TEER of the Transwell reached 400 to 500 Ω·cm². The 10% FBS-containing DMEM medium was exchanged for a calcium-free DMEM medium, and an oligosaccharide containing 1-kestose was added at a concentration of 1 wt.% to the upper layer. The medium was exchanged for a calcium-free medium one hour or six hours later, and TEER was chronologically measured three times, every half hour (1-hour calcium (+) 1% kestose group, 6-hour calcium (+) 1% kestose group, n = 3). As the control group, a group in which culture was performed for one hour or six hours in a calcium-containing DMEM medium, and then in a calcium-free DMEM medium (1-hour calcium (+) negative control group, 6-hour calcium (+) negative control group, n = 3) and a group of cells that were cultured only in a calcium-containing DMEM medium (positive control group, n = 3) were prepared, and chronological changes in TEER were compared.

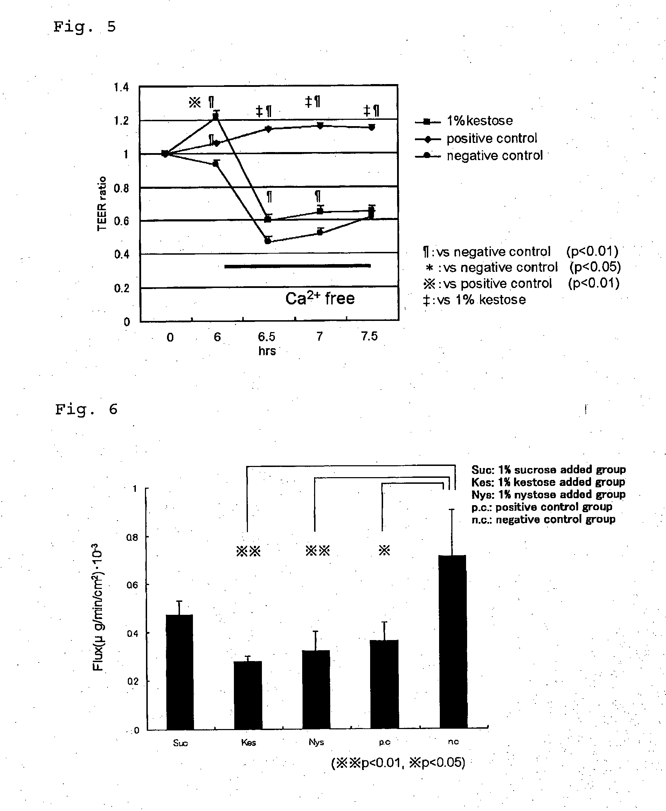

Hereinbelow, the results of measurement of TEER ratio in Example 4 are shown in Figures 4 and 5. Figure 4 shows the results of comparison among the 1-hour calcium (+) 1% kestose group, the 1-hour calcium (+) negative control group, and the positive control group. The 1-hour calcium (+) 1% kestose group exhibited a significant increase in TEER in comparison with the 1-hour calcium (+) negative control group and the positive control group in the TEER measurement conducted right before exchanging the medium for a calcium-free DMEM medium. Also, in a state in which the medium was exchanged for a calcium-free DMEM medium and 1-kestose was eliminated, there was a tendency that a decrease in TEER in the 1-hour calcium (+) 1% kestose group was inhibited in comparison with the 1-hour calcium (+) negative control group.

Next, Figure 5 shows the results of comparison among the 6-hour calcium (+) 1% kestose group, the 6-hour calcium (+) negative control group, and the positive control group. Similarly to Figure 4, the 6-hour calcium (+) 1% kestose group exhibited a significant increase in TEER in comparison with the 6-hour calcium (+) negative control group and the positive control group in the TEER measurement conducted right before exchanging the medium for a calcium-free DMEM medium. Similarly, in a state in which the medium was exchanged for a calcium-free DMEM medium and an oligosaccharide containing 1-kestose was eliminated, there was a tendency that a decrease in TEER in the 6-hour calcium (+) 1% kestose group was inhibited in comparison with the 6-hour calcium (+) negative control group.

The above observations confirmed that prior administration of 1-kestose had a preventive action on a decrease in the epithelial cell-cell adhesion, and 1-kestose exhibited an action of promoting the epithelial cell-cell adhesion in its presence.

### [Example 5: An experiment confirming the inhibitory effect on substance permeation against increased permeability of cell membrane caused by extracellular calcium deficiency]

In Example 5, the inhibitory effect of 1-kestose on substance permeation against increased permeability of cell membrane caused by extracellular calcium deficiency was confirmed by an experiment using cultured cells, including a comparison with other fructo-oligosaccharides.

An oligosaccharide containing 1-kestose at the same purity as the one used in Example 1 was used, and the cell culture was performed under the same conditions as Example 1.

The Caco-2 cells that were cultured until TEER of the Transwell reached 400 to 500 Ω·cm² were cultured for two hours after exchanging the 10% FBS-containing DMEM medium for a calcium-free colorless HBSS medium (GIBCO). Subsequently, a calcium-containing colorless HBSS medium (GIBCO) to which an oligosaccharide containing 1-kestose, sucrose, or nystose was added at a concentration of 1 wt.% was added to the upper layer. At the same time, Lucifer Yellow (MP Biomedical LLC.) was added to the upper layer at a concentration of 100 µM as a substance passing between the cells. After culturing for three hours, the HBSS medium in the lower layer was collected, and the residual concentration of Lucifer Yellow that had permeated from the upper layer to the lower layer was measured (1% kestose group, 1% sucrose group, and 1%
nystose group, n = 3). As the control group, a group of cells cultured only in a calcium-free HBSS medium (negative control group, n = 3) and a group in which culture was performed for two hours in a calcium-free HBSS medium, and then the medium was only exchanged for a calcium-containing HBSS medium (positive control group, n = 3) were prepared, and the amounts of substances that passed between the cells were compared.

Hereinbelow, the results of measurement of the residual concentration of Lucifer Yellow in the lower layer in Example 5 are shown in Figure 6. Compared to the negative control group, a significant reduction in the residual concentration was observed in the 1% kestose group, the 1% nystose group, and the positive control group. Also, compared to the positive control group, there was a tendency that the 1% kestose group and the 1% nystose group exhibited further reduced residual concentrations, and there was a tendency that the 1% kestose group showed the lowest residual concentration.

The above observations confirmed that 1-kestose had an inhibitory effect on the permeability of an allergenic protein and the like by promoting the epithelial cell-cell adhesion.

### [Example 6: An experiment confirming the effect on inhibition of a decrease in the epithelial cell-cell adhesion caused by the inflammatory cytokine IL-1β

In Example 6, the effect of 1-kestose on inhibition of a decrease in the epithelial cell-cell adhesion caused by an inflammatory cytokine was confirmed by an experiment using cultured cells.

An oligosaccharide containing 1-kestose at the same purity as the one used in Example 1 was used, and the cell culture was performed under the same conditions as Example 1.

To the upper layer of a DMEM medium in which the Caco-2 cells were cultured until TEER of the Transwell reached 400 to 500 Ω·cm², an oligosaccharide containing 1-kestose was added at a concentration of 1 wt.%. After culturing for 24 hours, the inflammatory cytokine IL-1β was added to the lower layer at a concentration of 10 ng/mL. TEER was measured 48 hours later and a ratio of the value thus measured to the initial measured value of TEER was calculated (1% kestose (+), IL-1β (+) group, n = 3). As the control group, a group in which only IL-1β was added (1% kestose (-), IL-1β (+) group, n = 3) or a group in which neither oligosaccharide containing 1-kestose nor IL-1β was added (1% kestose (-), IL-1β (-) group, n = 3) were prepared and TEER ratios were compared.

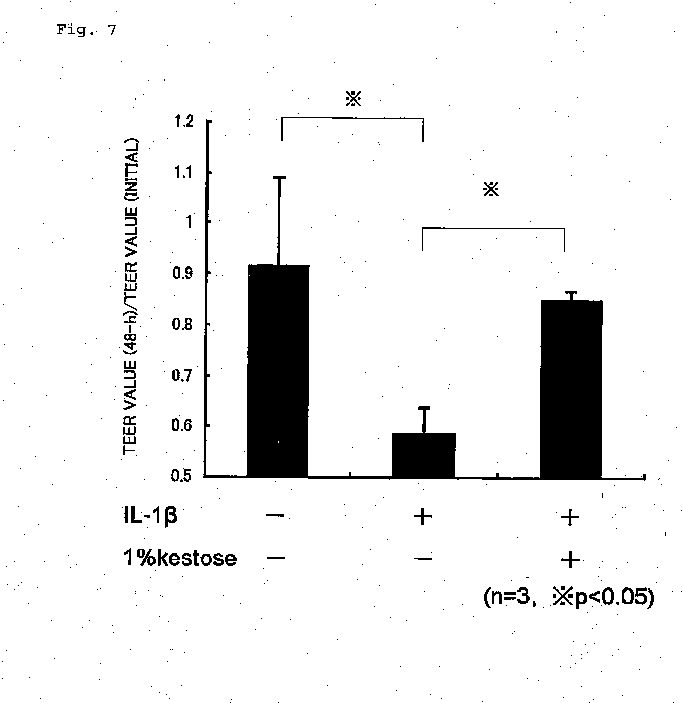

Hereinbelow, the results of measurement of TEER ratio in Example 6 are shown in Figure 7. The 1% kestose (-), IL-1β (-) group showed an average TEER ratio of 0.914 with a standard deviation of 0.031, while the 1% kestose (-), IL-1β (+) group showed an average TEER ratio of 0.585 with a standard deviation of 0.002 and the 1% kestose (+), IL-1β (+) group showed an average TEER ratio of 0.849 with a standard deviation of 0.0003. These results showed that a decrease of TEER, which is caused by IL-1β, was inhibited by approximately 26% by the addition of the oligosaccharide containing 1-kestose. These observations confirmed that 1-kestose had an effect of inhibiting a decrease in the epithelial cell-cell adhesion caused by topical inflammatory reaction.

The present invention repairs, or promotes the formation of, the tight junction protein between epithelial cells, which is assumed to be a cause of allergic diseases, thereby contributing to amelioration, treatment, and prevention of allergic diseases.

## Claims

1. 1-kestose and/or nystose and a divalent metal cation for use in ameliorating, treatment or prevention of allergic diseases, wherein 1-kestose and/or nystose and the divalent metal cation act as a cell-cell adhesion enhancer for activation of epithelial cells, and wherein the divalent metal cation is a calcium ion.

2. 1-kestose and/or nystose and a divalent metal cation for use according to claim 1, wherein 1-kestose and/or nystose is 1-kestose.

3. 1-kestose and/or nystose and a divalent metal cation for use according to any of claims 1 or 2, wherein the 1-kestose is an oligosaccharide containing 1-kestose at a purity of 95 wt.% or more, and the divalent metal cation is present in one part by weight or more per 10 parts by weight of the oligosaccharide containing 1-kestose.

4. 1-kestose and/or nystose and a divalent metal cation for use according to any of claims 1 to 3, wherein the epithelial cell is an intestinal epithelial cell.

5. 1-kestose and/or nystose and a divalent metal cation for use according to any of claims 1 to 4, wherein 1-kestose and/or nystose and a divalent metal cation are administered orally or parenterally.

## Patentansprüche

1. 1-Kestose und/oder Nystose und ein bivalentes Metallkation zur Verwendung in der Verbesserung, Behandlung oder Prävention von allergischen Erkrankungen, wobei 1-Kestose und/oder Nystose und das bivalente Metallkation als ein Zell-Zell-Adhäsions-Verstärker zur Aktivierung von Epithelzellen fungieren, und wobei das bivalente Metallkation ein Kalziumion ist.

2. 1-Kestose und/oder Nystose und ein bivalentes Metallkation zur Verwendung gemäß Anspruch 1, wobei 1-Kestose und/oder Nystose 1-Kestose ist.

3. 1-Kestose und/oder Nystose und ein bivalentes Metallkation zur Verwendung gemäß irgendeinem der Ansprüche 1 oder 2, wobei 1-Kestose ein Oligosaccharid, enthaltend 1-Kestose mit einer Reinheit von 95 Gew.-% oder mehr, ist, und das bivalente Metallkation mit 1 Gewichtsanteil oder mehr pro 10 Gewichtsanteile des Oligosaccharids enthaltend 1-Kestose vorhanden ist.

4. 1-Kestose und/oder Nystose und ein bivalentes Metallkation zur Verwendung gemäß irgendeinem der Ansprüche 1 bis 3, wobei die Epithelzelle eine intestinale Epithelzelle ist.

5. 1-Kestose und/oder Nystose und ein bivalentes Metallkation zur Verwendung gemäß irgendeinem der Ansprüche 1 bis 4, wobei 1-Kestose und/oder Nystose und ein bivalentes Metallkation oral oder parenteral verabreicht werden.

## Revendications

1. 1-kestose et/ou nystose et cation de métal divalent pour utilisation dans l'amélioration, le traitement ou la prévention de maladies allergiques, dans lesquels le 1-kestose et/ou le nystose et le cation de métal divalent agissent comme promoteur d'adhérence cellule-cellule pour l'activation des cellules épithéliales et dans lesquels le cation de métal divalent est un ion de calcium.

2. 1-kestose et/ou nystose et cation de métal divalent pour utilisation selon la revendication 1, dans lesquels le 1-kestose et/ou le nystose est ou sont du 1-kestose.

3. 1-kestose et/ou nystose et cation de métal divalent pour utilisation selon l'une quelconque des revendications 1 ou 2, dans lesquels le 1-kestose est un oligosaccharide contenant du 1-kestose d'une pureté de 95 % en poids ou plus et le cation de métal divalent est présent à raison d'une partie en poids ou plus par 10 parties en poids de l'oligosaccharide contenant du 1-kestose.

4. 1-kestose et/ou nystose et cation de métal divalent pour utilisation selon l'une quelconque des revendications 1 à 3, dans lesquels la cellule épithéliale est une cellule épithéliale intestinale.

5. 1-kestose et/ou nystose et cation de métal divalent pour utilisation selon l'une quelconque des revendications 1 à 4, dans lesquels le 1-kestose et/ou le nystose et un cation de métal divalent sont administrés par voie orale ou parentérale.
